# EUROPEAN PATENT SPECIFICATION

(11) **EP 0 957 836 B1**
(45) Date of publication and mention of the grant of the patent: **17.03.2004**
(21) Application number: 96938581.4
(22) Date of filing: 06.11.1996
(51) Int. Cl.: A61F 2/62

(54) **ADJUSTABLE ADAPTER FOR LOWER LEG PROSTHESIS**
EINSTELLBARER ADAPTER FÜR EINE UNTERSCHENKELPROTHESE
ADAPTATEUR REGLABLE POUR PROTHESE DE LA JAMBE INFERIEURE

(30) Priority: 07.11.1995 SE 9503944
(43) Date of publication of application: 24.11.1999
(73) Proprietor: GRAMTEC INNOVATION AB, S-511 56 Kinna (SE)
(72) Inventor: GRAMNÄS, Finn, S-511 56 Kinna (SE)
(74) Representative: Hammond, Andrew
(86) International application number: PCT/SE1996/001422
(87) International publication number: WO 1997/017042

(56) References cited:
- WO-A-93/17640
- US-A- 5 458 657

## Description

The present invention refers to an adjustable adapter for lower leg prostheses of the type described in the preamble of claim 1.

Prostheses for the knee, lower leg or foot are normally fastened to the bearer's bone using a prosthesis collar. To make the prosthesis adjustable at the desired angle and to make it possible to adj'ust it transversely to the prosthesis collar adapters providing such adjustability have previously been proposed.

SE-B-8602143-3 describes a lower leg prosthesis equipped with such an adapter which includes a prosthesis collar attachment member for attachment to the lower collar of the prosthesis. On the side facing the prosthesis collar the prosthesis collar attachment member is screwed to an adapter element including a little hemisphere around the screw shaft, which hemisphere is made rotatable against a corresponding spheroidal inner surface in an adapter housing which is attached to a lower leg prosthesis at its opposite end. At a distance from the hemisphere there is a concentrically attached disc against which the prosthesis collar attachment abuts. Further, there are external bosses on the adapter housing equipped with threaded penetrating holes at an angle to the central axis in which pinning screws can be screwed in from below so that their front ends press against the disc attached to the hemisphere thus pressing the adapter housing to different angular positions relative to the adapter housing. The prosthesis collar attachment element has a penetrating hole of significantly greater dimensions than the fastening screws in it whereby using a washer it is possible to attach the prosthesis collar attachment element in different sideways adjustable positions relative to the adapter housing. Using this design it is possible to achieve different angular positions without detaching the prosthesis, but because of the small radius of the sphere the angular variation is relatively limited. At the same time a sideways adjustment can be achieved but for this it is necessary that the whole prosthesis including the prosthesis collar be detached to reach the side adjustment screws, all of which makes fitting more difficult.

WO 93/17640 discloses an adapter for a prosthesis providing for angular and lateral adjustment of a prosthesis with respect to a prosthesis socket. The adapter is constructed so that these two adjustment functions are depending on each other.

Other types of similar adapters have the two adjustment functions separated to such a degree that the designs are large and unwieldy.

The purpose of the present invention is to provide an adjustable adapter for a lower leg prosthesis for which the above disadvantages are essentially eliminated, which has been achieved by giving the adapter the characteristics described in the characterising parts of the claims.

In the following the invention will be described in greater detail with reference to the exemplifications illustrated schematically in the attached figures.
Fig. 1 shows an exemplification of a lower leg prosthesis with included adapter according to the present invention in schematic cross section from the side.
Fig. 2 is a side view of the adapter according to Fig. 1.
Fig. 3 shows a blow-up view from the side of the adapter according to Fig. 2.
Fig. 4 is a blow-up view corresponding to Fig. 3 which shows the adapter's most important parts in section.
Fig. 5 shows an adapter head included in the adapter according to the invention in a view from above.
Fig. 6a-g show different adjustments of the adapter according to the invention in side views seen in section.

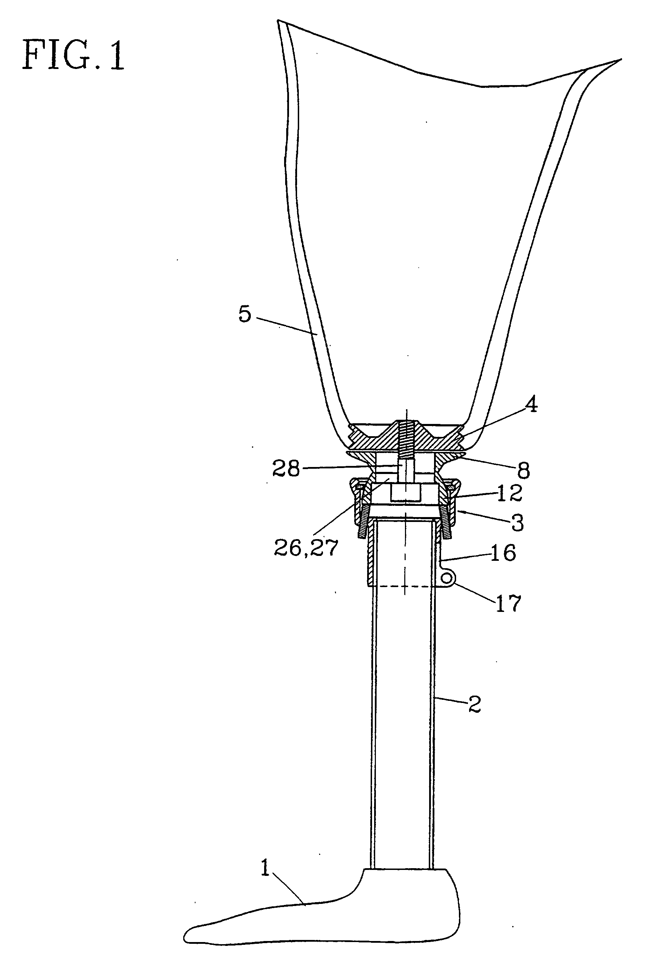

In Fig. 1 a prosthesis foot 1 with an adjacent long leg like element, preferably a tube 2, appropriately made of aluminum or another light strong material, is shown schematically. On the end facing away from the prosthesis foot the leg like element 2 is attached to an adapter 3 according to the invention, which is affixed to a prosthesis collar 5 using a prosthesis collar attachment member 4 that can be adjusted relative to the attachment end of the leg like element.

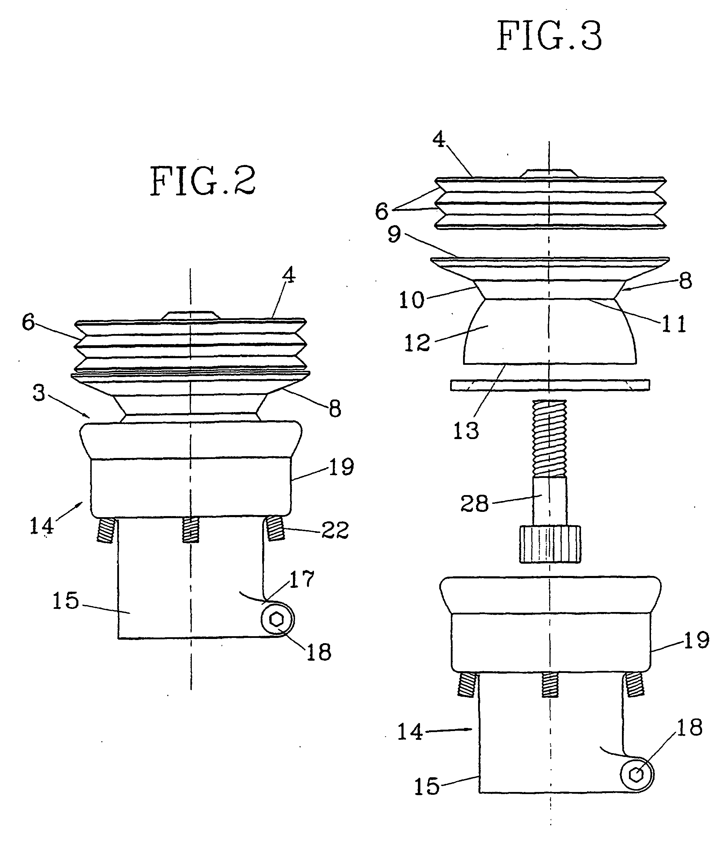

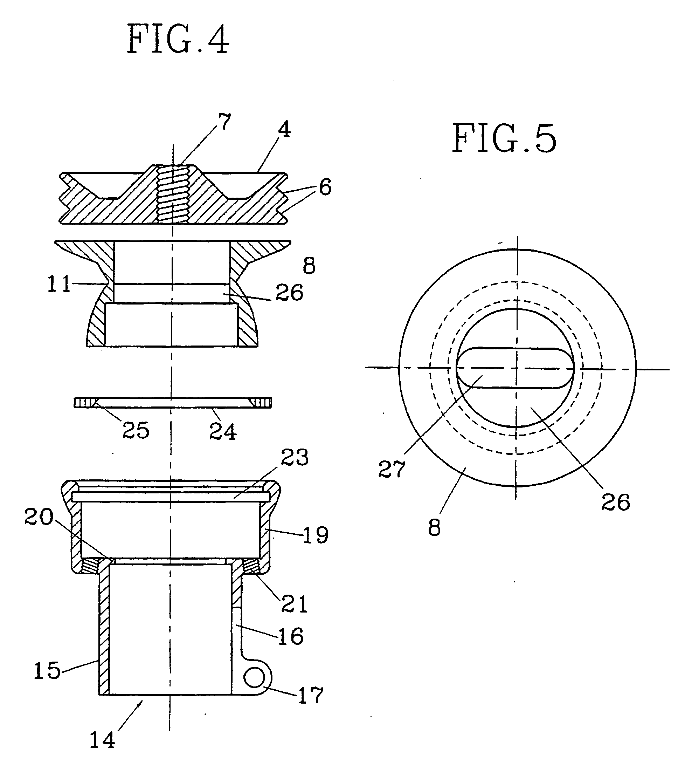

Fig. 2 to 4 show the adapter according to Fig. 1 on a larger scale from the side in a blow up view and in a blow up view in section, respectively. It is clear from the figures that the previously mentioned prosthesis collar attachment member 4 is included in the adapter 3, which member in the shown design consists of a circular disc with external grooves 6 which facilitate attachment of the member 4 to the prosthesis collar. In the shown design the disc has a central threaded hole 7 (see Fig. 1 and Fig. 4). For reasons to be given shortly, the prosthesis collar attachment member 4 or the central hole 7 can include an oblong hole (not shown). Further included in the adapter 3 is an adapter head 8 consisting of a collar with a circular ring shaped end surface 9 facing toward the prosthesis collar attachment member 4 and pressing against it. On the end surface 9 there is a conical section 10 which from a midline 11 is transformed to an essentially spherical concentric form 12. The spherical part 12 of the adapter ends in a circular ring shaped surface 13 which is mainly parallel to the adapter head's opposite end surface 9. Further there is an adapter housing 14 which includes a tube shaped section 15 designed to be attached to the tube like structure, for which purpose the under part of which has a slit 16 (see for example Fig. 1) while the material on either side of the slit 16 has attachment members 17 for a connector 18, for example a bolt, with which the end of the tube shaped part, which is somewhat elastic because of the slit, can be fastened to the upper end of the tube like structure 2. On the end facing away from the slit part of the tube shaped section 15 of the adapter housing 14 the tube like structure meets a bowl shaped sleeve 19 concentric with the tube shaped section 15 and of a larger diameter than the latter. The sleeve 19 and the tube shaped section meet each other at a ring shaped connector 20 attached essentially perpendicular to the adapter housing's central axis. Through that connector extend a number, preferably four, equidistant angled threaded holes 21 designed for adjustment means in the form of pinning screws 22 or the like. On the end of the collar 19 away from the tube shaped section 15 the collar has an encircling groove for a lock washer 24 with a hole 25 preferably of a form complementary to that of the spheroidal form 12 and with a contact line which, when assembled symmetrically, is in the middle of the spheroidal surface 12, that is, with its smaller opening turned axially out from the collar part when assembled, so that because of the increasing diameter of the spheroidal part 12 in the downward direction, after insertion into the sleeve 19 and attachment of the lock washer 24 the adapter head cannot be extracted from the sleeve but using the pinning screws it can be locked at a desired angle. The lock washer 24 is preferably equipped with a slit (not shown) which facilitates its insertion into the groove 23.

The inner diameter of the opening in the sleeve 19 is somewhat larger that the outer diameter of the end surface 13 of the adapter head's spheroidal part 12 so that it fits into the sleeve and at its ring shaped end surface presses against the pinning screws 22 in the adapter housing 14, as for instance is seen in Fig. 1 where the adapter head's middle part 11 is above the sleeve 19. By inserting the lock washer 24 into the groove 23 in the adapter housing 24 after inserting the adapter head 8 into it the lock washer prevents the adapter head from being withdrawn from the sleeve 19 while at the same time because of the spheroidal part 12 the adapter head can be set at an angle relative to the lengthwise axis of the adapter housing. The angled hole 25 in the lock washer 24 thus also contributes to making that angular positioning smooth and frictionless without scratching contact between the spheroidal part 12 and the edge of the hole 25.

In Fig. 4 it is clear from the section of the adapter head 8 that approximately at the level of the external middle part 11 it has an interior partition which has a penetrating oval hole 27 placed symmetrically on the center of the interior partition 26, as is clear from Fig. 5 in which the adapter head is shown in a top view.

For internal connection between the prosthesis collar attachment member 4 and the adapter head 8 a connector 28 is used, in the example shown in the form of a bolt through the oval hole 27 in the adapter head with its threaded shaft screwed into the threaded hole 7 in the prosthesis collar attachment member 4. Because the connector 28 has a part that cannot go through the oval hole 27, that is, in the present case the head of the bolt is larger in diameter that the width of the hole 27, the connector can be applied without its head going through the hole 27. Because that hole is oval it is still possible to attach the connector 28 in different positions along the length of the hole making adjustment along the length of the hole possible. With the alternative design of the prosthesis collar attachment member with an oblong hole instead of the centered hole 7 using a slide groove (not shown) in the prosthesis collar attachment member it is possible to use a nut as the connector 28 which is self locking along the length of the hole in the prosthesis collar attachment member 4. Thus the extent of the adjustability of the prosthesis adapter is further increased.

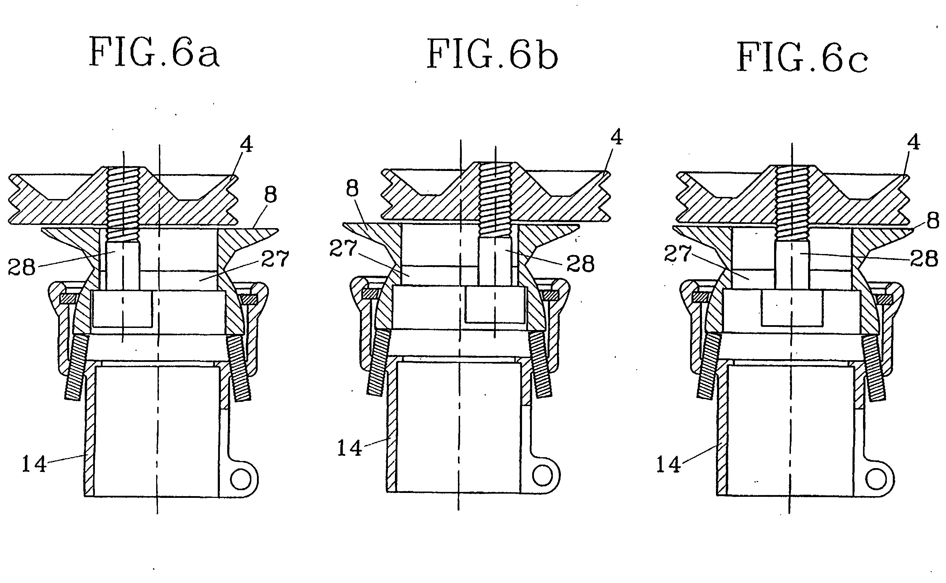

In Fig. 6a-g the adapter 3 according to the invention is shown in section in different adjustment positions between the prosthesis collar attachment member 4 and the adapter head 8 and between the adapter head 8 and the adapter housing 14. In this way a great variety of adjustment positions is made possible which further can be realised with an adapter that can be of a low and particularly compact design because of the large spheroidal part of the adapter head in the sleeve of the adapter housing.

Thus Fig. 6a shows how the adapter head 8 is adjusted so that its center line forms a direct extension of the center line of the adapter housing 14, that is, no angular displacement of the center line results. On the other hand the axis of the prosthesis collar attachment member 4 is shifted parallel with respect to the center line of the adapter head 8 and therefore to the center axis of the adapter housing. This has been achieved by shifting the connector 28 to its position, in the figure, furthest to the left in the oval hole 27 in the adapter head.

Also in Fig. 6b and 6c the adapter head 8 and the adapter housing are arranged with no angle between them while the prosthesis collar attachment member 4 in Fig. 6b is placed at its other maximally shifted end position and the connector 28 in Fig. 6c is placed centrally in the hole 27 so that the center line of the prosthesis collar attachment member 4 not only is parallel to but also coincides with the center line of the adapter housing 14 and of the adapter head 8.

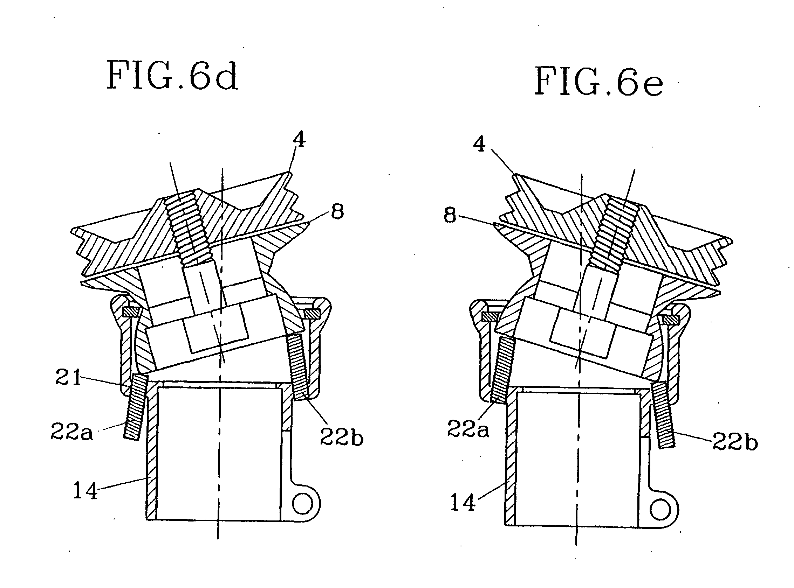

In Fig. 6d and 6e is shown how the prosthesis collar attachment member 4 and the adapter head 8 are in the same relative position as shown in Fig. 6c but where in both cases the adapter head 8 has been rotated in its seat in the adapter housing 14 relative to the latter so that their respective center lines form an angle. This relative angular adjustment has been achieved in Fig. 6d by unscrewing the front pinning screw 22a almost completely from its hole 21 while the back screw has been screwed in to a corresponding extent. Thus the back screw 22b in the figure lifts the right part of the adapter head 8 which, because of the spheroidal external surface placed inside the sleeve part of the adapter housing, tips in the sleeve and rests at an angle to the left in the figure with its own center axis and the prosthesis collar attachment member center axis maximally angled to the left relative to the center axis of the adapter housing.

In Fig. 6e the situation is the reverse, that is, the front pinning screw 22a has been screwed in maximally while the back one 22b has been screwed out. The angle of the coincident center lines for the prosthesis collar attachment member 4 and the adapter head 8 relative to the center axis of the adapter head 8 is as great as is the case in Fig. 6d but in this case the center line of the prosthesis collar attachment member 4 and the adapter head 8 is angled to the right relative to the center line of the adapter housing.

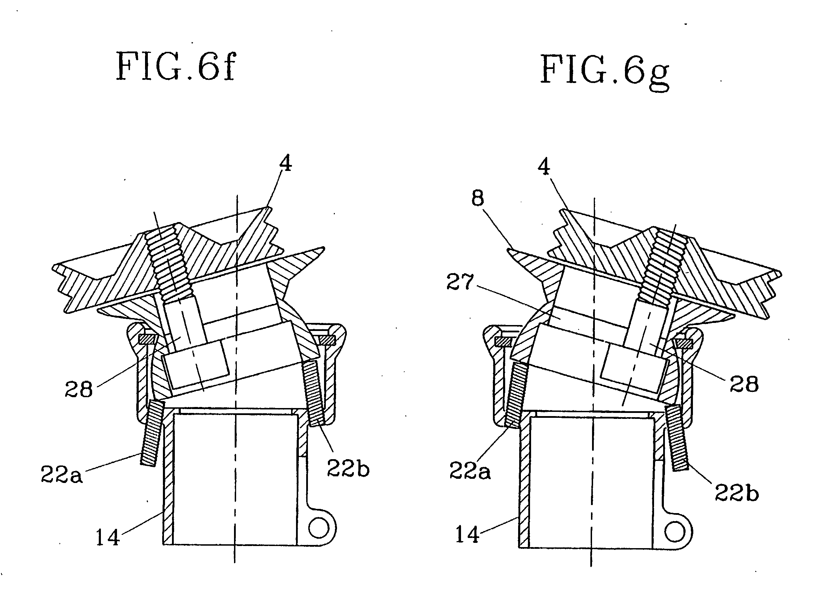

In Fig. 6f and 6g are finally shown two positions in which the adapter housing 14 and the adapter head 8 are angled relative to each other in the same way is in figures 6d and 6e where the angular adjustment has been made in the same way as in the two previously described cases. In addition the center axes of the prosthesis collar attachment member 4 and the adapter head 8 in the two figures 6f and 6g are shifted parallel relative to each other in the same way and by the same means as in figures 6a and 6b, respectively.

For simplicity in figures 6d to 6g how the adapter head 8 and the adapter housing 14 have been angled relative to each other has been shown only in the plane of the paper where only the front and back pinning screws 22a and 22b, respectively, have been screwed into or out from their neutral positions. Because the adapter housing 14 can also have more equidistant adjustment screws 22, preferably four, it is assumed that the angular adjustment can also be achieved in a plane perpendicular to the plane of the paper and/or in a combination of the planes, that is, the angle between the adapter housing and the adapter head, and thereby between the lower leg tube and the prosthesis collar attachment member 4, can be adjusted as desired to all angular positions while at the same time a sideways shift of the lower leg tube 2 and the prosthesis collar attachment member 4 can be obtained. Because the adapter head 8 has no fixed position relative to the adapter housing 14 the adapter head can also be rotated relative to the adapter housing 14 so that a parallel shift of the center axes of the prosthesis collar attachment member 4 and the adapter head 8 can be achieved in any desired direction perpendicular to the center axis of the adapter housing.

Using the design's large spheroidal adapter housing, which can be locked in position relative to the adapter housing in the way described and which has a built in sideways shift function, an extremely compact and therefore space saving design and a total adjustablity for an externally applied knee/lower leg/foot prosthesis is achieved.

The invention is not limited to the shown and described exemplifications but instead modifications are possible within the frame of the following patent claims.

## Claims

1. Adjustable adapter (3) for a lower leg prothesis (2), including an adapter housing (14) attachable to the lower leg prothesis (2), an adapter haead (8) having a part-spheroidal portion (12) moveably mounted in the adapter housing (14) and angularly adjustable by means of adjustment means (22) connected to the adapter housing (14), and a prothesis collar attachment member (4) which is sideways displaceable relative to the adapter head (8), said prothesis collar attachment member (4) is connected to a prothesis collar (5) and to the adapter housing (14),
**characterized in**
**that** the part-spheroidal portion (12) of the adapter head (8) in mounted position form an integrated unit with the adapter housing (14), wherein angular relationship between the part-spheroidal portion (12) and the adapter housing (14) is adjustable by means of said adjustment means (22) independant of the sideways displaceability and attachment of the adapter housing to the prothesis collar attachment member (4),
**that** in the adapter head (8) there is provided an elongated hole (27), through which a connector (28), connecting the prosthetic collar attachment member (4) and the adapter head (8), extends and which permit a transversal movement between the adapter head (8) and the prothesis collar attachment (4) independant of the angular adjustability between the adapter housing (14) and the adapter head (8).

2. Adapter according to claim 1,
**characterized in**
**that** the part-spheroidal portion (12) of the adapter head (8) is designed to pass into a waist protion (11) and then into a substantially conically widening portion (10), which terminates with a plane end surface (9) intended to contact the prothesis collar attachment member (4).

3. Adapter according to claim 1,
**characterized in**
**that** the part-spheroidal portion (12) of the adapter head (8) is fixed in the adapter housing by means of a preferably openable lock washer (24).

4. Adapter according to claim 1,
**characterized in**
**that** one end surface (9) of the adapter housing (8) and a ring-shaped end surface (13) of the part-spheroidal portion (12) are substantially parallel, and that the adjustment means (22) of the adapter housing (8) is arranged to act upon said ring-shaped end surface (13) of the part-spheroidal portion (12).

5. Method of mounting an adjustable adapter (3) for lower leg prothesis (2) including an adapter housing (14) and an adapter head (8) having a part-spheroidal portion (12) rotatably mounted therein and which by means of adjustment means (22) connected to the adapter housing is angularly adjustable with respect to this,
**characterized in**
**that** the part-spheroidal portion (12) of the adapter head (8) is inserted in a socket-provided (19) part of the adapter housing, that an openable lock washer (24) is placed about a waist (11) between the part-spheroidal portion (12) of the adapter head (8) and a conically widening connection portion (10) connected therewith,
**that** the adapter head (8) is inserted into the socket (19) so far that the waist will be located just opposite a ring groove (23) in the socket,
**that** the lock washer (24) is snapped into the ring groove (23), and that the adjustment means (22) is tightened to such an extent that the part-spheroidal portion is pressed in contact against the lock washer.

## Patentansprüche

1. Verstellbarer Adapter (3) für eine Unterschenkelprothese (2), einschließend ein Adaptergehäuse (14), das an der Unterschenkelprothese (2) anbringbar ist, einen Adapterkopf (8) mit einem Abschnitt in Form eines Sphäroidteils (12), der beweglich im Adaptergehäuse (14) montiert ist und dessen Winkeleinstellung durch Stellmittel (22), die mit dem Adaptergehäuse (14) verbunden sind, erfolgen kann, und ein Prothesenmanschetten-Befestigungsglied (4), das relativ zum Adapterkopf (8) seitwärts verschiebbar und mit einer Prothesenmanschette (5) und dem Adaptergehäuse (14) verbunden ist,
**dadurch gekennzeichnet,**
**dass** der Abschnitt in Form eines Sphäroidteils (12) des Adapterkopfs (8) in montierter Stellung in das Adaptergehäuse (14) integriert ist, wobei der Winkel, den der Abschnitt in Form eines Sphäroidteils (12) und das Adaptergehäuse (14) in Bezug zueinander bilden, unabhängig von der seitlichen Verschiebbarkeit und der Befestigung des Adaptergehäuses am
Prothesenmanschetten-Befestigungsglied (4) durch die Stellmittel (22) einstellbar ist,
**dass** sich im Adapterkopf (8) ein Langloch (27) befindet, durch das sich ein Verbindungsstück (28), das das Prothesenmanschetten-Befestigungsglied (4) und den Adapterkopf (8) miteinander verbindet, erstreckt und das unabhängig von der Einstellbarkeit des Winkels zwischen dem Adaptergehäuse (14) und dem Adapterkopf (8) eine Querbewegung zwischen dem Adapterkopf (8) und dem Prothesenmanschetten-Befestigungsglied (4) ermöglicht.

2. Adapter nach Anspruch 1,
**dadurch gekennzeichnet,**
**dass** der Abschnitt in Form eines Sphäroidteils (12) des Adapterkopfs (8) zunächst in einen schmalen Mittelabschnitt (11) und dann in einen sich im Wesentlichen konisch erweiternden Abschnitt (10) übergeht, der durch eine ebene Endfläche (9) begrenzt wird, die mit dem Prothesenmanschetten-Befestigungsglied (4) in Berührung treten soll.

3. Adapter nach Anspruch 1,
**dadurch gekennzeichnet,**
**dass** der Abschnitt in Form eines Sphäroidteils (12) des Adapterkopfs (8) durch eine vorzugsweise bewegliche Sicherungsscheibe (24) im Adaptergehäuse befestigt ist.

4. Adapter nach Anspruch 1,
**dadurch gekennzeichnet,**
**dass** eine Endfläche (9) des Adapterkopfs (8) und eine ringförmige Endfläche (13) des Abschnitts in Form eines Sphäroidteils (12) im Wesentlichen parallel zueinander verlaufen und dass das Stellmittel (22) des Adaptergehäuses (14) auf die ringförmige Endfläche (13) des Abschnitts in Form eines Sphäroidteils (12) einwirkt.

5. Verfahren zur Montage eines verstellbaren Adapters (3) für eine Unterschenkelprothese (2), einschließend ein Adaptergehäuse (14) und einen Adapterkopf (8) mit einem Abschnitt in Form eines Sphäroidteils (12), der drehbar im Adaptergehäuse (14) montiert ist und dessen Winkeleinstellung in Bezug auf das Adaptergehäuse durch Stellmittel (22), die mit dem Adaptergehäuse verbunden sind, erfolgen kann,
**dadurch gekennzeichnet,**
**dass** der Abschnitt in Form eines Sphäroidteils (12) des Adapterkopfs (8) in einen mit einer Muffe versehenen Teil (19) des Adaptergehäuses eingeführt wird, dass eine bewegliche Sicherungsscheibe (24) um einen schmalen Mittelabschnitt (11) herum angeordnet ist, der sich zwischen dem Abschnitt in Form eines Sphäroidteils (12) des Adapterkopfs (8) und einem sich konisch erweiternden Verbindungsabschnitt (10) des Adapterkopfs befindet,
**dass** der Adapterkopf (8) so weit in die Muffe (19) eingeführt wird, dass sich der schmale Mittelabschnitt genau gegenüber einer Ringnut (23) in der Muffe befindet,
**dass** die Sicherungsscheibe (24) in der Ringnut (23) einrastet und dass das Stellmittel (22) in einem solchen Maße festgezogen wird, dass der Abschnitt in Form eines Sphäroidteils gegen die Sicherungsscheibe gedrückt wird und so mit dieser in Berührung tritt.

## Revendications

1. Adaptateur ajustable (3) destiné à une prothèse (2) de la partie inférieure de la jambe, comprenant un boîtier (14) d'adaptateur fixable à la prothèse (2) de la partie inférieure de la jambe, une tête (8) d'adaptateur présentant une portion partiellement sphéroïdale (12) montée mobile dans le boîtier (14) d'adaptateur et réglable angulairement par des moyens de réglage (22) reliés au boîtier (14) d'adaptateur, et un élément (4) de fixation de l'emboîture de la prothèse, qui peut être déplacé latéralement par rapport à la tête (8) de l'adaptateur, ledit élément (4) de fixation de l'emboîture de la prothèse étant relié à une emboîture (5) de prothèse et au boîtier (14) d'adaptateur,
***caractérisé***
***en ce que*** la portion partiellement sphéroïdale (12) de la tête (8) de l'adaptateur; en position montée, forme une unité intégrée avec le boîtier (14) d'adaptateur, dans laquelle la relation angulaire entre la portion partiellement sphéroïdale (12) et le boîtier (14) d'adaptateur est réglable au moyen desdits moyens de réglage (22) indépendamment de la possibilité de déplacement latéral et de la fixation du boîtier d'adaptateur à l'élément (4) de fixation de l'emboîture de prothèse,
***en ce que*,** dans la tête (8) de l'adaptateur, est ménagé un trou longitudinal (27), à travers lequel un connecteur (28), reliant l'élément (4) de fixation de l'emboîture de prothèse et la tête (8) de l'adaptateur, s'étend et qui permet un mouvement transversal entre la tête (8) de l'adaptateur et la fixation (4) de l'emboîture de prothèse, indépendamment de l'ajustabilité angulaire entre le boîtier (14) d'adaptateur et la tête (8) de l'adaptateur.

2. Adaptateur selon la revendication 1, ***caractérisé en ce que*** la portion partiellement sphéroïdale (12) de la tête (8) de l'adaptateur est conçue pour se transformer en portion rétrécie (11) puis en portion (10) s'élargissant de manière sensiblement conique, qui se termine avec une surface terminale plane (9) destinée à être en contact avec l'élément (4) de fixation de l'emboîture de prothèse.

3. Adaptateur selon la revendication 1, ***caractérisé en ce que*** la portion partiellement sphéroïdale (12) de la tête (8) de l'adaptateur est fixée dans le boîtier d'adaptateur au moyen d'une rondelle (24) d'arrêt de préférence ouvrable.

4. Adaptateur selon la revendication 1, ***caractérisé en ce qu***'une surface terminale (9) de la tête (8) de l'adaptateur et une surface terminale annulaire (13) de la portion partiellement sphéroïdale (12) sont sensiblement parallèles, et ***en ce que*** les moyens de réglage (22) de la tête (8) de l'adaptateur sont prévus pour agir sur ladite surface terminale annulaire (13) de la portion partiellement sphéroïdale (12).

5. Procédé de montage d'un adaptateur ajustable (3) destiné à une prothèse (2) de la partie inférieure de la jambe, comprenant un boîtier (14) d'adaptateur et une tête (8) d'adaptateur ayant une portion partiellement sphéroïdale (12) montée rotative dans celui-ci et qui, par des moyens de réglage (22) reliés au boîtier d'adaptateur, est réglable angulairement par rapport à lui,
***caractérisé***
***en ce que*** la portion partiellement sphéroïdale (12) de la tête (8) de l'adaptateur est insérée dans une portion équipée d'un manchon (19) du boîtier d'adaptateur,
***en ce qu***'une rondelle (24) d'arrêt ouvrable est placée autour d'un rétrécissement (11) entre la portion partiellement sphéroïdale (12) de la tête (8) de l'adaptateur et une portion (10) de raccord s'élargissant de manière conique raccordée à celui-ci,
***en ce que*** la tête (8) de l'adaptateur est insérée dans le manchon (19) d'une distance telle que le rétrécissement soit situé de manière juste opposée à une rainure annulaire (23) dans le manchon,
***en ce que*** la rondelle (24) d'arrêt est encliquetée dans la rainure annulaire (23),
et ***en ce que*** les moyens de réglage (22) sont serrés de telle sorte que la portion partiellement sphéroïdale soit poussée en contact contre la rondelle d'arrêt.
